# EUROPÄISCHE PATENTSCHRIFT

(11) **EP 3 907 500 B1**
(45) Veröffentlichungstag und Bekanntmachung des Hinweises auf die Patenterteilung: **12.11.2025**
(21) Anmeldenummer: 21170814.4
(22) Anmeldetag: 28.04.2021
(51) Int. Cl.: G01N 25/48, G01N 33/487

(54) **VERFAHREN UND SYSTEM ZUR ANALYSE VON BIOLOGISCHEM MATERIAL**
METHOD AND SYSTEM FOR ANALYZING BIOLOGICAL MATERIAL
PROCÉDÉ ET SYSTÈME D'ANALYSE DE MATÉRIEL BIOLOGIQUE

(30) Priorität: 08.05.2020 DE 102020112538
(43) Veröffentlichungstag der Anmeldung: 10.11.2021
(73) Patentinhaber: Netzsch-Gerätebau GmbH, 95100 Selb (DE)
(72) Erfinder: Taubmann, Rebekka, 95100 Selb (DE)
(74) Vertreter: Isarpatent

(56) Entgegenhaltungen:
- WO-A1-2017/066800
- KR-A- 20200 011 874
- US-A1- 2019 003 995
- US-A1- 2020 029 858
- WONHEE LEE ET AL: "Development and applications of chip calorimeters as novel biosensors", NANOBIOSENSORS IN DISEASE DIAGNOSIS, 20 April 2012 (2012-04-20), pages 17, XP055146018, DOI: 10.2147/NDD.S26438

## Beschreibung

Die vorliegende Erfindung betrifft ein Verfahren und ein System zur Analyse durch dynamische Differenzkalorimetrie (DSC), von biologischem Material, insbesondere Blut, Urin, Schweiß oder Hautgewebe, sowie eine Verwendung eines derartigen Systems.

Analysen zum Erkennen von Krankheiten mittels dynamischer Differenzkalorimetrie (DSC) werden typischerweise im medizinischen Bereich in der Forschung angewendet. Der Aufbau der notwendigen Messapparaturen beinhaltet dabei überwiegend Apparaturen, die in Forschungslaboren üblich und einsetzbar sind. So sind einige Komponenten, beispielsweise Messvorrichtungen und Energieversorgung, immobil ausgebildet, da sie beispielsweise über ein Stromkabel mit dem Stromnetz des Gebäudes verbunden sind. Zudem liegen die Kosten der Messapparaturen in einer Höhe, die Institutionen außerhalb der Forschung und Privatpersonen nicht wirtschaftlich aufbringen können und deshalb auf die Anschaffung verzichten. Daraus folgt, dass insbesondere die Wartezeiten für benötigte Analysen sehr lange sind und die Kapazitäten für Analysen pro Zeiteinheit gering sind.

Ferner zeigen die Untersuchungen aus den Forschungslaboren, dass anhand von Analysen mittels dynamischer Differenzkalorimetrie Krankheiten erkannt und Krankheitsverläufe überwacht werden können. Dabei bieten derartige Analysen im Vergleich zu herkömmlichen Blutanalysen, wie sie alternativ ausgeführt werden können, einen sehr viel detaillierteren Aufschluss über den Gesundheitszustand eines Patienten.

Es sind Vorrichtungen und Verfahren bekannt, wie sie typischerweise zu Forschungszwecken Anwendung finden.

Die US 2019/0003995 A1 beschreibt eine dynamische Differenzkalorimeter-Vorrichtung zum Erkennen von Krankheiten und Überwachen der therapeutischen Wirksamkeit durch das Erkennen wärmebeständiger Varianten von Proteinen und/oder Stoffwechselprodukten in biologischen Proben.

Die WO 2017/066800 A1 beschreibt Verfahren zum Charakterisieren und/oder Vorhersagen von Risiko verbunden mit einer biologischen Probe unter Verwendung von thermischen Stabilitätsprofilen.

Der Artikel Wonhee Lee et al.: "Development and applications of chip calorimeters as novel biosensors", NANOBIOSENSORS IN DISEASE DIAGNOSIS, 20. April 2012, Seite 17, XP055146018,DOI: 10.2147/NDD.S26438 beschreibt die Verwendung eines kalometrischen Wegwerfsensorens zur Untersuchung biologischen Materials.

Es ist Aufgabe der vorliegenden Erfindung, Möglichkeiten zur kostengünstigeren, einfacheren und schnelleren Erkennung von Krankheiten zu schaffen.

Erfindungsgemäß wird diese Aufgabe jeweils durch die Gegenstände der unabhängigen Ansprüche gelöst.

Ein erster Aspekt der Erfindung ist ein Verfahren nach Anspruch 1 zur Analyse durch dynamische Differenzkalorimetrie (DSC), von biologischem Material, insbesondere Blut, Urin, Schweiß oder Hautgewebe.

Ein zweiter Aspekt der Erfindung ist ein System nach Anspruch 6 zur Analyse durch dynamische Differenzkalorimetrie (DSC), von biologischem Material, insbesondere Blut, Urin, Schweiß oder Hautgewebe.

Eine der vorliegenden Erfindung zugrunde liegende Idee besteht darin, Gesundheitszustände von Patienten schnell erkennen und Verläufe der Gesundheitszustände detailliert verfolgen zu können. Dabei kann die Erfindung sowohl als zusätzliches Hilfsmittel für Ärzte als auch als eigenständiges Instrument zum Verfolgen des täglichen Gesundheitszustandes von Privatpersonen dienen. Da es insbesondere nicht speziell geschulten Personen ermöglicht werden soll ohne lange Wartezeit und zu vertretbaren Kosten ihren Gesundheitszustand abschätzen zu lassen, kann das vorliegend vorgeschlagene System als mobiles System ausgebildet sein. Ferner können das System oder Komponenten des Systems auch zu anderen kommunikationsfähigen Gegenständen kompatibel und/oder verbunden sein, sodass das System oder Komponenten des Systems beispielsweise am Körper tragbar sind.

Vorteilhafte Ausführungsformen und Weiterbildungen ergeben sich aus den auf die unabhängigen Ansprüche rückbezogenen Unteransprüchen sowie aus der Beschreibung unter Bezugnahme auf die Figuren.

Gemäß der Erfindung werden beim Abschätzen des Gesundheitszustandes zusätzlich zu den Messwerten ferner Datensätze mit medizinischen Informationen berücksichtigt. Somit kann der Gesundheitszustand basierend auf umfangreichen Datensätzen aus weiteren Quellen, welche über die Messdaten des primären Verfahrens hinausgehen, noch präziser abgeschätzt werden. Relevante Datensätze könnten dabei vergangene medizinische Informationen des Patienten und/oder Daten aus anderen Messmethoden, die der Patient zusätzlich zu dem vorliegenden Verfahren anwendet, sein.

Diese Datensätze umfassen auch vergleichbare Messwerte anderer Patienten und/oder auf die vergleichbaren Messwerte angewendete Behandlungsmaßnahmen, und werden durch kommunikatives Koppeln der Auswerteeinrichtung mit einem Datenverarbeitungsgerät die Datensätze an die Auswerteeinrichtung übermittelt. Auf diese Weise können durch Ärzte oder sonstiges Fachpersonal validierte Gesundheitszustände, welche auf vergleichbaren Messwerten anderer Patienten beruhen, bei dem Abschätzen des Gesundheitszustandes helfen eine zuverlässigere Aussage zu erhalten. Ferner können Behandlungsmaßnahmen gezielt empfohlen werden, da diese beispielsweise bereits bei vergleichbaren Messwerten erfolgreich angewendet wurden.

Gemäß einer Weiterbildung umfassen die Datensätze Vitalparameter des Patienten, insbesondere Blutzuckergehalt, Blutdruck, Herzfrequenz und ähnliches, welche durch kommunikatives Koppeln der Auswerteeinrichtung mit diagnostischen Geräten, insbesondere Blutzucker-/Blutdruckmessgeräten oder ähnlichen, und/oder Computer-Hardware, insbesondere Fitness-Trackern, an die Auswerteeinrichtung übermittelt werden, wobei die Vitalparameter im Wesentlichen zu derselben Zeit wie die Probe mit biologischem Material erhoben werden. Somit können zum Abschätzen des Gesundheitszustandes Datensätze herangezogen werden, die üblicherweise mittels einfach zu bedienenden Geräten von nicht speziell geschulten Personen selbstständig erhoben werden können und folglich eine bessere Gesamteinschätzung des Gesundheitszustandes des Patienten ermöglichen. Die Auswerteeinrichtung kann entweder kontinuierlich oder durch jeweilige Freigabe an den entsprechenden Geräten mit den Datensätzen gespeist werden.

Gemäß einer weiteren Ausführungsform des Verfahrens führt die Messeinrichtung ein weiteres thermisches Analyseverfahren zur Messung von abgegebener oder aufgenommener Wärmemenge des biologischen Materials während eines thermischen Prozesses durch. Durch die Analyse von biologischem Material in Abhängigkeit von der Temperatur kann der Gesundheitszustand des Patienten präziser abgeschätzt werden, da die thermische Analyse detaillierter und somit aufschlussreicher als eine gewöhnliche Blutanalyse ist.

Erfindungsgemäß wird der Sensor nach dem Schritt des Abschätzens entsorgt. Auf diese Weise können notwendige Hygiene-Richtlinien eingehalten und Fehler beim Abschätzen des Gesundheitszustandes durch verunreinigte Sensoren verringert werden, da der Sensor bei jeder Anwendung stets neu und beispielsweise separat verpackt ist und die schützende Verpackung erst entfernt wird, wenn der Sensor verwendet wird.

Gemäß einer weiteren Ausführungsform kommuniziert die Auswerteeinrichtung mit einer Vorrichtung, welche vorzugsweise als Spiegel, Fernseher und/oder Computer-Hardware, insbesondere PC, Smartphone, Smartwatch und/oder Fitness-Tracker, ausgebildet ist, über jeweilige Kommunikationsschnittstellen. Somit kann die Auswerteeinrichtung von der Vorrichtung Datensätze, insbesondere auf dem Patienten zur Verfügung stehenden Datenspeichern, wie eine Cloud oder ähnliches, oder auf Servern von Kliniken oder Krankenkassen gespeicherte Datensätze, abrufen oder an diese übermitteln ohne selbst mit einem Server oder dergleichen kommunikativ gekoppelt zu werden. Auf diese Weise können die persönlichen Daten des Patienten leichter geschützt werden.

Gemäß einer Weiterbildung wird beim Abschätzen des Gesundheitszustandes eine auf der Vorrichtung oder auf einem Server gespeicherte zweite Anwendungssoftwareinstanz ausgeführt, welche ein präziseres Abschätzen des Gesundheitszustandes als die erste Anwendungssoftwareinstanz der Auswerteeinrichtung ermöglicht, und/oder wird der Gesundheitszustand auf einem Display der Vorrichtung visualisiert oder audiovisualisiert. Mittels der zweiten Anwendungssoftwareinstanz kann die Anwendungskomplexität und die Informationsausgabe im Vergleich zu der ersten Anwendungssoftwareinstanz gesteigert werden, sodass Fachpersonal wie beispielsweise Ärzte zusätzlich die in der Messeinrichtung gemessenen Messwerte angezeigt bekommen und darauf basierend den von der Auswerteeinrichtung abgeschätzten Gesundheitszustand validieren können oder eine umfangreichere Abschätzung des Gesundheitszustandes angezeigt bekommen als ungeschulte Personen, die eine weniger differenzierte Abschätzung des Gesundheitszustandes auf Basis der ihnen kaum interpretierbaren Messwerte angezeigt bekommen.

Gemäß einer weiteren Ausführungsform umfasst das Verfahren ferner einen Schritt des Steuerns kommunikationsfähiger Gebäudeeinrichtungen, insbesondere Heizungen, Gebäudebelüftungen und/oder Wecker, zur Unterstützung von Behandlungsmaßnahmen für den Patienten in Abhängigkeit von dem abgeschätzten Gesundheitszustand. Auf diese Weise kann die unmittelbare Umgebung des Patienten, insbesondere die Raumtemperatur, -luftfeuchtigkeit und/oder Helligkeit automatisch an empfohlene Behandlungsmaßnahmen angepasst werden. Ferner können durch die Gebäudeeinrichtungen Erinnerungen oder Informationen, beispielsweise zu anstehender Medikamenteneinnahme, an den Patienten visuell und/oder akustisch übermittelt werden.

Gemäß der Erfindung umfasst die Auswerteeinrichtung ferner eine Kommunikationsschnittstelle, welche dazu ausgebildet ist, eine Kommunikationsverbindung zwischen der Auswerteeinrichtung und einem externen Kommunikationsteilnehmer aufzubauen und zum Abschätzen des Gesundheitszustandes Datensätze mit medizinischen Informationen von dem externen Kommunikationsteilnehmer an die Auswerteeinrichtung zu übermitteln.

Somit ist die Auswerteeinrichtung in der Lage, den Gesundheitszustand basierend auf zusätzlichen Datensätzen aus dem externen Kommunikationsteilnehmer, welche über die Messdaten des primären Verfahrens hinaus gehen, noch präziser abzuschätzen. Zusätzliche Datensätze könnten dabei vergangene medizinische Informationen des Patienten und/oder Daten aus anderen Messmethoden, die der Patient zusätzlich zu dem vorliegenden Verfahren anwendet, sein.

Der externe Kommunikationsteilnehmer ist als Datenverarbeitungsgerät ausgebildet und über die Kommunikationsschnittstelle mit der Auswerteeinrichtung kommunikativ gekoppelt, wobei die Datensätze insbesondere vergleichbare Messwerte anderer Patienten und/oder auf die vergleichbaren Messwerte angewendete Behandlungsmaßnahmen umfassen. Auf diese Weise können auf dem Datenverarbeitungsgerät gespeicherte durch Ärzte oder sonstiges Fachpersonal validierte Gesundheitszustände, welche auf vergleichbaren Messwerten anderer Patienten beruhen, der Auswerteeinrichtung zur Verfügung stehen, um eine zuverlässigere Aussage zu erhalten. Ferner können Behandlungsmaßnahmen gezielt auf dem Display dargestellt sein, da diese beispielsweise bereits bei vergleichbaren Messwerten erfolgreich angewendet wurden.

Gemäß einer weiteren Weiterbildung des Systems ist der externe Kommunikationsteilnehmer als diagnostisches Gerät, insbesondere als Blutzucker-/Blutdruckmessgerät oder ähnliches, und/oder als Computer-Hardware, insbesondere als Fitness-Tracker, ausgebildet und über die Kommunikationsschnittstelle mit der Auswerteeinrichtung kommunikativ gekoppelt, wobei die Datensätze vorzugsweise Vitalparameter des Patienten, insbesondere Blutzuckergehalt, Blutdruck, Herzfrequenz und ähnliches, umfassen. Somit sind für die Auswerteeinrichtung zum Abschätzen des Gesundheitszustandes Datensätze heranziehbar, die üblicherweise mittels einfach zu bedienenden Geräten von nicht speziell geschulten Personen selbstständig erhoben werden können und folglich eine bessere Gesamteinschätzung des Gesundheitszustandes des Patienten ermöglichen. Die Auswerteeinrichtung kann entweder kontinuierlich oder durch jeweilige Freigabe an den entsprechenden Geräten mit den Datensätzen gespeist sein.

Gemäß einer weiteren Ausführungsform des Systems ist die Messeinrichtung ferner dazu ausgebildet, ein thermisches Analyseverfahren zur Messung von abgegebener oder aufgenommener Wärmemenge des biologischen Materials während eines thermischen Prozesses zu ermöglichen. Indem die Messeinrichtung in der Lage ist, die Analyse von biologischem Material in Abhängigkeit von der Temperatur zu ermöglichen, ist der Gesundheitszustand des Patienten präziser abschätzbar, da die thermische Analyse detaillierter und somit aufschlussreicher als eine gewöhnliche Blutanalyse ist.

Gemäß einer weiteren Ausführungsform des Systems ist die Messeinrichtung ein Einwegprodukt. Auf diese Weise können notwendige Hygiene-Richtlinien eingehalten und Fehler beim Abschätzen des Gesundheitszustandes durch verunreinigte Messeinrichtungen verringert werden, da die Messeinrichtung bei jeder Anwendung stets neu und beispielsweise separat verpackt ist und die schützende Verpackung erst entfernt wird, wenn die Messeinrichtung verwendet wird.

Gemäß einer Weiterbildung des Systems ist der externe Kommunikationsteilnehmer vorzugsweise als Spiegel, Fernseher, Server und/oder Computer-Hardware, insbesondere PC, Smartphone, Smartwatch und/oder Fitness-Tracker, zur Visualisierung oder Audiovisualisierung des Gesundheitszustandes ausgebildet. Somit ist die Auswerteeinrichtung ausgebildet, Datensätze, insbesondere auf Servern von Kliniken oder Krankenkassen abgespeicherte Datensätze, von dem externen Kommunikationsteilnehmer abzurufen oder an diesen zu übermitteln ohne selbst mit einem Server oder dergleichen kommunikativ gekoppelt zu sein. Auf diese Weise sind die persönlichen Daten des Patienten leichter schützbar.

Gemäß einer weiteren Weiterbildung des Systems weist der externe Kommunikationsteilnehmer oder ein über die Kommunikationsschnittstelle gekoppelter Server eine zweite Anwendungssoftwareinstanz auf, welche ein präziseres Abschätzen des Gesundheitszustandes als die erste Anwendungssoftwareinstanz der Auswerteeinrichtung ermöglicht. Somit kann die erste Anwendungssoftwareinstanz hinsichtlich Anwendungskomplexität und Informationsausgabe entsprechend der zu erwartenden Fachkenntnisse des Anwenders eingerichtet sein. Mittels der zweiten Anwendungssoftwareinstanz kann die Anwendungskomplexität und die Informationsausgabe im Vergleich zu der ersten Anwendungssoftwareinstanz gesteigert sein, sodass für Fachpersonal wie beispielsweise Ärzte zusätzlich die Messwerte anzeigbar sind und darauf basierend der abgeschätzten Gesundheitszustand validierbar ist oder eine umfangreichere Abschätzung des Gesundheitszustandes angezeigt ist als für ungeschulte Personen, die zu einer weniger differenzierten Abschätzung des Gesundheitszustandes auf Basis der ihnen kaum interpretierbaren Messwerte in der Lage sind.

Gemäß einer weiteren Ausführungsform des Systems sind die Messeinrichtung, die Auswerteeinrichtung und das Display von einem Gehäuse, insbesondere einem gemeinsamen Gehäuse, zumindest teilweise umgeben. Somit sind das System oder zumindest Komponenten des Systems mobil tragbar ausgebildet.

Die obigen Ausführungsformen und Weiterbildungen lassen sich, sofern sinnvoll, beliebig miteinander kombinieren. Der Umfang der Erfindung wird nur durch die Ansprüche bestimmt.

Die vorliegende Erfindung wird nachfolgend anhand von Ausführungsbeispielen unter Bezugnahme auf die beiliegenden Figuren der Zeichnungen näher erläutert. Von den Figuren zeigen:
- Fig. 1: ein Ablaufdiagramm eines Verfahrens zur Analyse durch dynamische Differenzkalorimetrie (DSC), von biologischem Material, insbesondere Blut, Urin, Schweiß oder Hautgewebe, gemäß einem Ausführungsbeispiel der Erfindung;
- Fig. 2: einen Ausschnitt eines Ablaufdiagramms für ein weiteres Ausführungsbeispiel der Schritte Senden der Messwerte und Abschätzen des Gesundheitszustandes des Verfahrens gemäß dem Ausführungsbeispiel nach Fig. 1;
- Fig. 3: eine schematische Darstellung eines Systems zur Analyse durch dynamische Differenzkalorimetrie (DSC), von biologischem Material, insbesondere Blut, Urin, Schweiß oder Hautgewebe, gemäß einem Ausführungsbeispiel der Erfindung;
- Fig. 4: eine schematische Seitenansicht der Messeinrichtung aus Fig. 3 gemäß einem weiteren Ausführungsbeispiel.

In den Figuren der Zeichnung sind gleiche, funktionsgleiche und gleich wirkende Elemente, Merkmale und Komponenten - sofern nichts anderes ausgeführt ist - jeweils mit denselben Bezugszeichen versehen.

Obwohl vorliegend spezifische Ausführungsformen und Weiterbildungen dargestellt und beschrieben sind, ist die Erfindung nicht hierauf beschränkt.

Die beiliegenden Figuren sollen ein weiteres Verständnis von Ausführungsformen der Erfindung vermitteln und dienen im Zusammenhang mit der Beschreibung der Erklärung von Prinzipien und Konzepten der Erfindung. Andere Ausführungsbeispiele und viele der genannten Vorteile ergeben sich im Hinblick auf die Zeichnungen. Die Zeichnungen sind lediglich als schematische Zeichnungen zu verstehen und die Elemente der Zeichnungen sind nicht notwendigerweise maßstabsgetreu zueinander dargestellt. Richtungsangebende Terminologie wie etwa "oben", "unten", "links", "rechts", "über", "unter", "horizontal", "vertikal", "vorne", "hinten" und ähnliche Angaben werden lediglich zu erläuternden Zwecken verwendet und dienen nicht der Beschränkung der Allgemeinheit auf spezifische Ausgestaltungen wie in den Figuren gezeigt.

Gestrichelte Linien in den Figuren der Zeichnungen verdeutlichen, dass die Verbindungen zwischen den die gestrichelten Linien verbindenden Komponenten nicht zwingend physischen Kontakt miteinander haben müssen, sondern gleichermaßen drahtlos zueinander gekoppelt sein können.

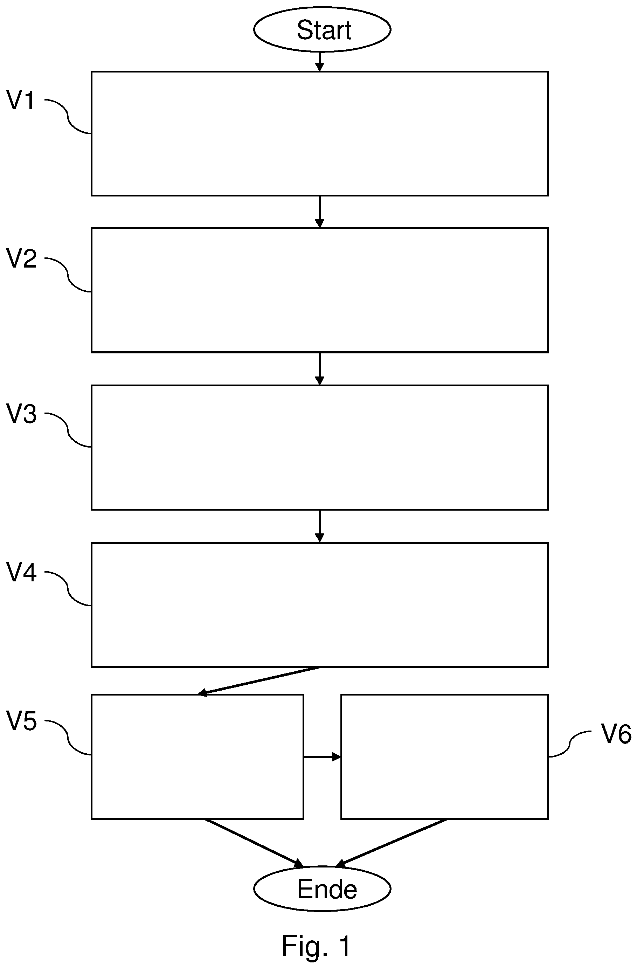

Fig. 1 zeigt ein Ablaufdiagramm eines Verfahrens zur Analyse durch dynamische Differenzkalorimetrie (DSC), von biologischem Material, insbesondere Blut, Urin, Schweiß oder Hautgewebe tierischen oder menschlichen Ursprungs, gemäß einem Ausführungsbeispiel der Erfindung. Das Verfahren umfasst die Schritte Einbringen V1 einer Probe 4, Erfassen V2 von Messwerten, Senden V3 der Messwerte an eine Auswerteeinrichtung 5, Abschätzen V4 eines Gesundheitszustandes eines Patienten und Visualisieren oder Audiovisualisieren V5 des Gesundheitszustandes.

Das Einbringen V1 einer Probe 4 mit biologischem Material des Patienten auf einen Sensor 3 einer Messeinrichtung 2 kann dabei durch mehrere Möglichkeiten ausgeführt werden. Dabei kann das biologische Material auf den Sensor 3 eingebracht werden, indem das biologische Material direkt auf den Sensor 3 oder in einen Behälter 14, zum Beispiel einen Tiegel oder vergleichbares, eingebracht wird, wobei der Behälter 14 den Sensor 3 kontaktiert oder in dem Sensor 3 enthalten ist.

Beispielsweise kann als biologisches Material Blut des Patienten, welches mittels einer Lanzette oder einer ähnlichen Stecheinrichtung aus dem Patienten hervorgebracht wurde, auf einen Chip, in den Behälter 14 oder direkt auf den Sensor 3 aufgebracht werden. Denkbar ist zudem, dass die Stecheinrichtung automatisch durch Freigabe des Patienten oder in vorgegebenen Zeitabständen Blut freisetzt, sodass dieses auf den Sensor 3 eingebracht werden kann. Des Weiteren kann die Probe 4 Urin des Patienten aufweisen und mittels einer Pipette, insbesondere einer Einweg-Pipette auf den Sensor 3 eingebracht werden. Ferner können beispielsweise auch Schweiß oder Hautgewebe mit geeigneten Handhabungsmitteln oder anderweitig als biologisches Material eingebracht werden. Alternativ kann das biologische Material passiv auf den Sensor 3 eingebracht werden, beispielsweise durch bereitgestellte Öffnungen/Kanäle, welche das biologische Material konstruktiv durch Schwerkraft auf dem Sensor 3 platzieren. Die Wahl des biologischen Materials zur Analyse ist nicht auf die genannten Beispiele beschränkt, sondern kann weitere biologische Materialien umfassen, welche medizinisch verwertbare Informationen zum Gesundheitszustand des Patienten ermöglichen.

In der Messeinrichtung 2 werden die Messwerte erfasst V2. Dazu führt die Messeinrichtung 2 ein thermisches Analyseverfahren zur Messung von abgegebener oder aufgenommener Wärmemenge des biologischen Materials während eines thermischen Prozesses durch.

Dieses thermische Analyseverfahren wird erfindungsgemäß zumindest als dynamische Differenzkalorimetrie durchgeführt. Darüber hinaus können ebenso eine Thermogravimetrie oder eine simultane Thermoanalyse zum Einsatz kommen, wodurch eine Messung des Massenverlustes der Probe 4 und/oder eine Infrarotspektroskopie von durch das Erhitzen der Probe 4 entstehenden Gasen möglich wird. Weiterhin sind darüber hinaus andere im medizinischen Bereich bekannte Analyseverfahren anwendbar.

Unabhängig von dem angewendeten Analyseverfahren können die Messwerte auch Messwerte einer bekannten Referenz 13 umfassen, wobei die Referenz 13 parallel zu der Probe 4 das Analyseverfahren durchläuft und somit als Vergleich dient.

Beim Schritt des Sendens V3 der Messwerte an die Auswerteeinrichtung 5, die mit der Messeinrichtung 2 kommuniziert, kann die Kommunikation sowohl kabellos, beispielsweise mittels Bluetooth oder WLAN, als auch drahtgebunden, beispielsweise mittels LAN oder USB, ausgeführt werden. Dabei können die Auswerteeinrichtung 5 und die Messeinrichtung 2 entweder direkt durch korrespondierende Kommunikationsschnittstellen oder über ein Datenverarbeitungsgerät, insbesondere ein PC, ein Smartphone oder vergleichbare digitale Kommunikationsgeräte, kommunizieren. Die Kommunikationsform zwischen der Messeinrichtung 2 und dem möglichen Datenverarbeitungsgerät sowie zwischen dem möglichen Datenverarbeitungsgerät und der Auswerteeinrichtung 5 muss nicht identisch sein. Ferner kann die Auswerteeinrichtung 5 mit einer Vorrichtung, welche vorzugsweise als Spiegel, Fernseher und/oder Computer-Hardware, insbesondere PC, Smartphone, Smartwatch und/oder Fitness-Tracker, ausgebildet ist, über jeweilige Kommunikationsschnittstellen kommunizieren.

In einem weiteren Schritt V4 wird der Gesundheitszustand des Patienten anhand von den Gesundheitszustand charakterisierenden Datenstrukturen auf Basis der Messwerte mittels der Auswerteeinrichtung 5, auf der eine erste Anwendungssoftwareinstanz 6a ausgeführt wird, abgeschätzt. Die den Gesundheitszustand charakterisierenden Datenstrukturen können bestimmte Datenpunkte sein, welche beispielsweise während der dynamischen Differenzkalorimetrie aufgezeichnet werden. Dabei erfahren die Probe 4 und die Referenz 13, die beide in einem im Wesentlichen identischen Behälter 14, beispielsweise einem Tiegel, oder auf im Wesentlichen identischen Sensoren 3 analysiert werden, dieselbe thermische Einwirkung durch Temperierelemente 12. Infolge der Wärmekapazität der Probe 4 bzw. der Referenz 13 und exothermen oder endothermen Prozessen, wie etwa Schmelzen oder Verdampfen, kann es zwischen der Probe 4 und der Referenz 13 zu Temperaturdifferenzen und somit verschiedenen Messwerten für dieselbe Messgröße kommen. Je nachdem zu welchem Zeitpunkt im Analyseverfahren Unterschiede zwischen der Probe 4 und der Referenz 13 auftreten und je nachdem wie groß diese Unterschiede ausfallen ergeben sich Datenstrukturen, die als charakteristisch für einen bestimmten Gesundheitszustand erachtet werden.

Ferner kann beim Abschätzen V4 des Gesundheitszustandes eine auf der Vorrichtung oder auf einem Server gespeicherte zweite Anwendungssoftwareinstanz 6b ausgeführt werden, welche ein präziseres Abschätzen des Gesundheitszustandes als die erste Anwendungssoftwareinstanz 6a der Auswerteeinrichtung 5 ermöglicht. Alternativ oder zusätzlich kann der Gesundheitszustand auf einem Display der Vorrichtung visualisiert oder audiovisualisiert werden.

Die Ausgestaltung der Anwendungssoftwareinstanzen 6a, 6b kann beispielsweise über vier Ausgestaltungsebenen differenziert werden. Dabei werden die Ausgabemöglichkeiten der Anwendungssoftwareinstanzen 6a, 6b an die zu erwartende Anwenderzielgruppe, insbesondere deren anzunehmende medizinischen Kenntnisse, angepasst. Beispielhaft seien vier Anwenderzielgruppen genannt, Privatpersonen ohne medizinische Kenntnisse, Apotheken, Haus- und Fachärzte, Krankenhäuser & sonstige medizinische Laboreinrichtungen, deren medizinische Kenntnisse in der Reihenfolge der Nennung zunehmen. Anhand der vier Ausgestaltungsebenen kann der jeweiligen Anwenderzielgruppe eine für sie verständliche Darstellung des abgeschätzten Gesundheitszustandes des Patienten visualisiert oder audiovisualisiert werden. Somit ist es möglich eigenständig, beispielsweise zuhause, die Probe 4 in die Messeinrichtung 2 einzubringen und die Messwerte an einen Experten, wie etwa einen Arzt, in der Ferne zu übermitteln, um dessen umfangreichere Ausgabemöglichkeiten der zweiten Anwendungssoftwareinstanz 6b, welche beispielsweise auf dessen Server in der Arztpraxis gespeichert ist, und seine fachmännische Einschätzung zu dem visualisierten Gesundheitszustand zu nutzen.

Optional kann die Messeinrichtung 2 nach dem Schritt des Abschätzens V4 entsorgt werden. Ein Schritt des Reinigens der Messeinrichtung 2 wird somit entbehrlich, da die Messeinrichtung 2 bei jeder Anwendung stets neu und beispielsweise separat verpackt ist und die schützende Verpackung erst entfernt wird, wenn die Messeinrichtung 2 verwendet wird.

Weiterhin umfasst das Verfahren ein Visualisieren oder Audiovisualisieren V5 des abgeschätzten Gesundheitszustandes sowie des Verlaufs des Gesundheitszustandes über einen bestimmten vergangenen Zeitraum auf einem Display 7. Dabei können dem Display 7 die benötigten Informationen von der Auswerteeinrichtung drahtgebunden oder drahtlos übermittelt werden.

Darüber hinaus können die Messwerte und/oder der abgeschätzte Gesundheitszustand lokal auf einem Datenträger oder auf einem Server, insbesondere in einer Cloud gespeichert werden. Außerdem können die Messwerte und/oder der abgeschätzte Gesundheitszustand mit Ärzten, Kliniken, Apotheken, Krankenkassen, Herstellern der Geräte, welche das erfindungsgemäße Verfahren durchführen, und/oder anderen Geräten, welche das erfindungsgemäße Verfahren durchführen, ausgetauscht werden.

Ferner kann das Verfahren einen Schritt des Steuerns V6 kommunikationsfähiger Gebäudeeinrichtungen, insbesondere Heizungen, Gebäudebelüftungen und/oder Wecker, zur Unterstützung von Behandlungsmaßnahmen für den Patienten in Abhängigkeit von dem abgeschätzten Gesundheitszustand umfassen.

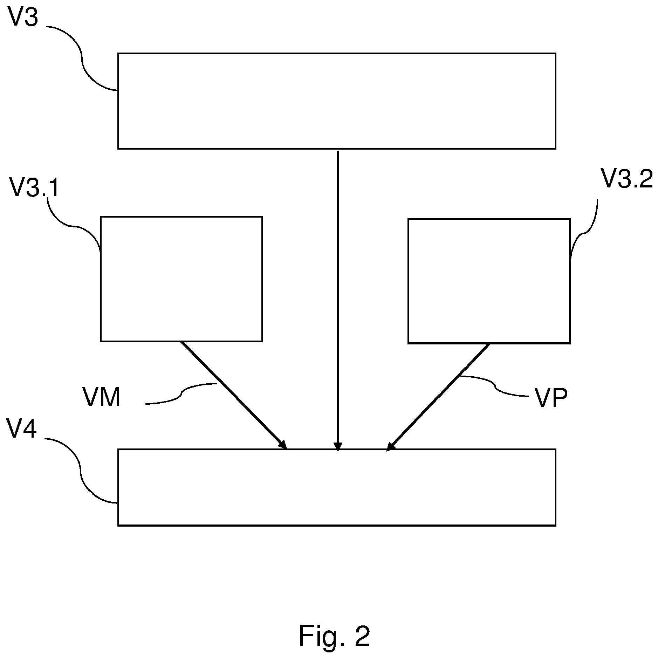

Fig. 2 zeigt einen Ausschnitt eines Ablaufdiagramms für ein weiteres Ausführungsbeispiel der Schritte Senden V3 der Messwerte und Abschätzen V4 des Gesundheitszustandes des Verfahrens gemäß dem Ausführungsbeispiel nach Fig. 1.

In den erfindungsgemäßen Verfahrenschritten nach Fig. 2 werden beim Abschätzen V4 des Gesundheitszustandes zusätzlich zu den Messwerten, welche durch die Messeinrichtung 2 bereitgestellt werden, ferner Datensätze mit medizinischen Informationen berücksichtigt, um möglichst viele Anhaltspunkte für die charakterisierenden Datenstrukturen vorzusehen. Diese Datensätze können medizinische Informationen aus mindestens zwei verschiedenen Bereichen aufweisen. Erfindungsgemäß umfassen die Datensätze vergleichbare Messwerte VM anderer Patienten und/oder auf die vergleichbaren Messwerte angewendete Behandlungsmaßnahmen. Hierbei werden durch kommunikatives Koppeln V3.1 der Auswerteeinrichtung 5 mit einem Datenverarbeitungsgerät, insbesondere einem PC, einem Smartphone oder vergleichbaren digitalen Kommunikationsgeräten, die Datensätze an die Auswerteeinrichtung 5 übermittelt.

Zusätzlich können die Datensätze Vitalparameter VP des Patienten, insbesondere Blutzuckergehalt, Blutdruck, Herzfrequenz und Ähnliches, umfassen. Dazu wird die Auswerteeinrichtung 5 mit diagnostischen Geräten, insbesondere Blutzucker/Blutdruckmessgeräten oder ähnlichen, und/oder Computer-Hardware, insbesondere Fitness-Trackern, kommunikativ gekoppelt V3.2. Über die bestehende Koppelung der Auswerteeinrichtung 5 mit den diagnostischen Geräten können die Datensätze an die Auswerteeinrichtung 5 übermittelt werden, wobei die Vitalparameter VP im Wesentlichen zu derselben Zeit wie die Probe 4 mit biologischem Material erhoben werden. Darüber hinaus oder alternativ können die in den Datensätzen enthaltenen medizinischen Informationen zumindest teilweise durch manuelle Eingabe des Patienten beispielsweise in die Auswerteeinrichtung 5, in das Datenverarbeitungsgerät oder das diagnostische Gerät bereitgestellt werden.

Alternativ oder zusätzlich können die Datensätze ferner Trink- und Essverhalten des Patienten umfassen, wobei diese Informationen durch manuelle Eingabe des Patienten in die Auswerteeinrichtung 5 oder über eine Koppelung derselben mit einem Datenverarbeitungsgerät, welches diese Informationen beinhaltet, eingegeben werden können.

Insofern kann die Auswerteeinrichtung 5 nicht nur mit einem der genannten externen Kommunikationsteilnehmer 9, d. h. dem Datenverarbeitungsgerät oder einem diagnostischen Gerät kommunikativ gekoppelt werden, sondern auch mit mehreren externen Kommunikationsteilnehmern 9, insbesondere dem Datenverarbeitungsgerät und den diagnostischen Geräten sowie weiteren nicht explizit aufgeführten externen Kommunikationsteilnehmern.

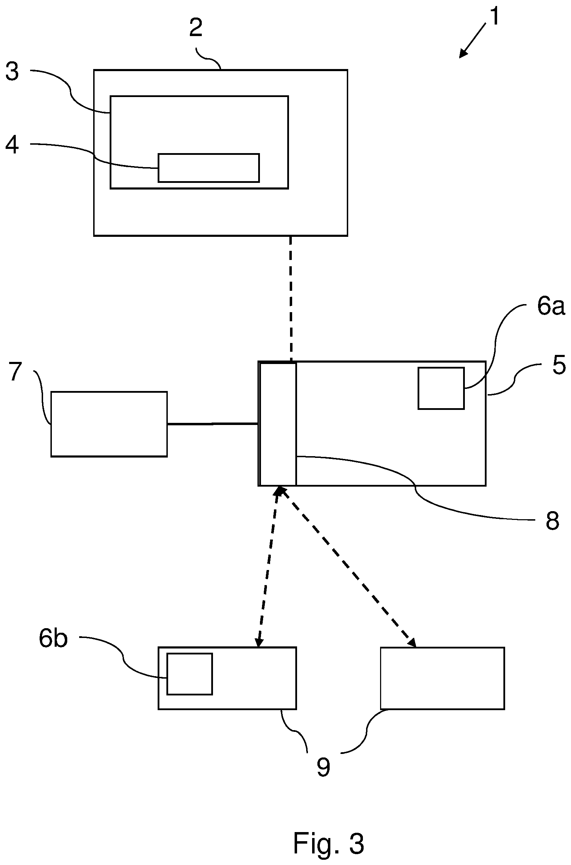

Fig. 3 zeigt eine schematische Darstellung eines Systems 1 zur Analyse durch dynamische Differenzkalorimetrie (DSC), von biologischem Material, insbesondere Blut, Urin, Schweiß oder Hautgewebe tierischen oder menschlichen Ursprungs, gemäß einem Ausführungsbeispiel der Erfindung. Das System 1 weist dabei eine Messeinrichtung 2, eine Auswerteeinrichtung 5 und ein Display 7 auf.

Die Messeinrichtung 2 weist einen Sensor 3 auf, der eine Probe 4 mit biologischem Material eines Patienten beinhaltet und dazu ausgebildet ist, Messwerte an die Auswerteeinrichtung 5 zu senden.

Zum Einbringen der Probe 4 mit biologischem Material in den Sensor 3 kann der Sensor 3 derart ausgebildet sein, dass das biologische Material beispielsweise mittels Einweg-Pipette oder anderen Handhabungsmitteln in die Messeinrichtung 2 einbringbar ist. Zur automatischen Aufnahme der Probe 4 mit biologischem Material, insbesondere Blut oder Schweiß, kann die Messeinrichtung 2 Öffnungen/Kanäle aufweisen, durch welche das biologische Material zum Beispiel konstruktiv durch Schwerkraft auf dem Sensor 3 platzierbar ist.

Darüber hinaus kann die Messeinrichtung 2 eine Energieversorgung (nicht dargestellt) umfassen, beispielsweise einen lokalen Energiespeicher, eine elektronische Verbindung zu einem energieführenden Gerät, dessen Energiequelle mitgenutzt wird, oder einen Stromnetzanschluss. In am Körper getragenen Ausführungsbeispielen kann durch den Einsatz von thermoelektrischen Generatoren, welche die Abwärme des Körpers zur Erzeugung von Energie nutzen, die Energieversorgung bereitgestellt sein.

In einem weiteren Ausführungsbeispiel kann die Messeinrichtung 2 ein Einwegprodukt sein. Dazu ist die Messeinrichtung 2 bevorzugt kompatibel austauschbar zu den anderen Komponenten des Systems 1 ausgebildet, insbesondere zu der Auswerteeinrichtung 5.

Die Auswerteeinrichtung 5 weist eine erste Anwendungssoftwareinstanz 6a auf und ist dazu ausgebildet, die Messwerte zu empfangen und einen Gesundheitszustand des Patienten anhand von den Gesundheitszustand charakterisierenden Datenstrukturen auf Basis der Messwerte abzuschätzen. Die erste Anwendungssoftwareinstanz 6a kann beispielsweise als Softwareprogramm oder Applikation auf der Auswerteeinrichtung 5 gespeichert sein.

Die Energieversorgung der Auswerteeinrichtung 5 kann der Energieversorgung der Messeinrichtung 2 entsprechen. In Ausführungsbeispielen in denen die Messeinrichtung 2 mit der Auswerteeinrichtung 5 elektronisch, elektrisch oder anderweitig energietransferierend gekoppelt ist kann eine gemeinsame Energieversorgung für die Messeinrichtung 2 und die Auswerteeinrichtung 5 vorgesehen sein.

Ferner umfasst die Auswerteeinrichtung 5 eine Kommunikationsschnittstelle 8, welche dazu ausgebildet ist, eine Kommunikationsverbindung zwischen der Auswerteeinrichtung 5 und einem externen Kommunikationsteilnehmer 9 aufzubauen. Darüber hinaus ist die Kommunikationsschnittstelle in der Lage, zum Abschätzen des Gesundheitszustandes Datensätze mit medizinischen Informationen von dem externen Kommunikationsteilnehmer 9 an die Auswerteeinrichtung 5 zu übermitteln. Der externe Kommunikationsteilnehmer 9 kann dabei mindestens in drei verschiedenen Gerätekategorien ausgebildet sein, wobei die Auswerteeinrichtung in der Lage ist, zu mehreren externen Kommunikationsteilnehmern 9, insbesondere mehreren aus verschiedenen Gerätekategorien, eine Kommunikationsverbindung aufzubauen.

Der externe Kommunikationsteilnehmer 9 ist erfindungsgemäß zumindest als Datenverarbeitungsgerät, insbesondere als Server oder Datenspeicher, beispielsweise einer Klinik oder Krankenkasse, ausgebildet und über die Kommunikationsschnittstelle 8 mit der Auswerteeinrichtung 5 kommunikativ gekoppelt, wobei die Datensätze insbesondere vergleichbare Messwerte anderer Patienten und/oder auf die vergleichbaren Messwerte angewendete Behandlungsmaßnahmen umfassen.

Zusätzlich kann der externe Kommunikationsteilnehmer 9 als diagnostisches Gerät, insbesondere als Blutzucker/Blutdruckmessgerät oder ähnliches, und/oder als Computer-Hardware, insbesondere als Fitness-Tracker, ausgebildet sein. Des Weiteren kann der externe Kommunikationsteilnehmer 9 über die Kommunikationsschnittstelle 8 mit der Auswerteeinrichtung 5 kommunikativ gekoppelt sein, wobei die Datensätze vorzugsweise Vitalparameter des Patienten, insbesondere Blutzuckergehalt, Blutdruck, Herzfrequenz und ähnliches, umfassen.

Ferner kann der externe Kommunikationsteilnehmer 9 vorzugsweise als Spiegel, Fernseher, Server und/oder Computerhardware, insbesondere PC, Smartphone, Smartwatch und/oder Fitness-Tracker, zur Visualisierung oder Audiovisualisierung des Gesundheitszustandes ausgebildet sein.

Die Koppelung der Auswerteeinrichtung 5 mit den externen Kommunikationsteilnehmern 9 zur Datenübertragung über die jeweiligen Kommunikationsschnittstellen 8 kann drahtgebunden, beispielsweise USB, LAN, oder kabellos, beispielsweise WLAN oder Bluetooth, vorgesehen sein. Dabei kann die Koppelung der Auswerteeinrichtung 5 mit jedem der externen Kommunikationsteilnehmer 9 individuell und unabhängig von der Art der Koppelung zu anderen externen Kommunikationsteilnehmern 9 sein und ist nicht auf eine Art der Koppelung zwischen der Auswerteeinrichtung 5 und den externen Kommunikationsteilnehmern 9 beschränkt.

Zusätzlich zu der ersten Anwendungssoftwareinstanz 6a der Auswerteeinrichtung 5 kann der externe Kommunikationsteilnehmer 9 oder ein über die Kommunikationsschnittstelle 8 gekoppelter Server eine zweite Anwendungssoftwareinstanz 6b aufweisen, welche ein präziseres Abschätzen des Gesundheitszustandes als die erste Anwendungssoftwareinstanz 6a der Auswerteeinrichtung 5 ermöglicht. Die Anwendungssoftwareinstanzen 6a, 6b können, wie in dem Ausführungsbeispiel nach Fig. 1 beschrieben, eingerichtet sein, sodass vier verschiedene Anwendungsebenen zum Abschätzen und Visualisieren des Gesundheitszustandes des Patienten je nach den zu erwartenden medizinischen Kenntnissen einer Anwenderzielgruppe möglich sind.

Ferner können die Messeinrichtung 2, die Auswerteeinrichtung 5 und das Display 7 von einem Gehäuse, insbesondere einem gemeinsamen Gehäuse, zumindest teilweise umgeben sein. Des Weiteren können Komponenten des Systems 1, insbesondere die Messeinrichtung 2 und die Auswerteeinrichtung 5, derart ausgebildet sein, dass das System 1 oder Komponenten des Systems 1 tragbar, insbesondere am menschlichen Körper tragbar sind, und/oder mit am Körper tragbaren Geräten koppelbar sind.

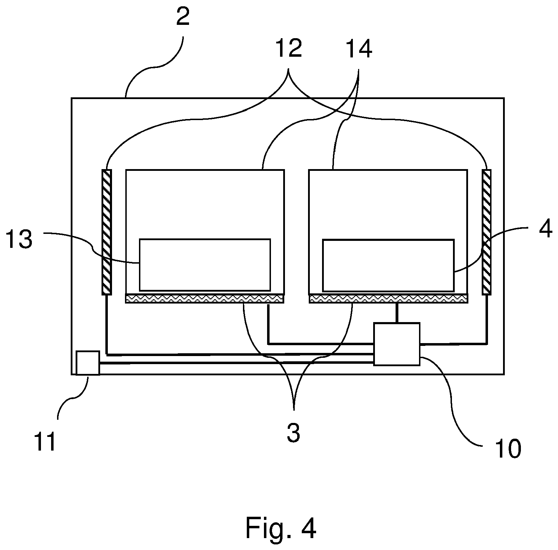

Fig. 4 zeigt eine schematische Seitenansicht der Messeinrichtung 2 aus Fig. 3 gemäß einem weiteren Ausführungsbeispiel. Diese Messeinrichtung 2 korrespondiert im Wesentlichen mit der Messeinrichtung 2, wie sie in dem Ausführungsbeispiel nach Fig. 3 beschrieben ist.

Ferner kann die Messeinrichtung 2 dazu ausgebildet sein, ein weiteres thermisches Analyseverfahren zur Messung von abgegebener oder aufgenommener Wärmemenge des biologischen Materials während eines thermischen Prozesses zu ermöglichen. Dabei kann die Messeinrichtung 2 beispielsweise Temperierelemente 12 aufweisen, mittels welcher die Probe 4 und eine Referenz 13 erhitzt und/oder gekühlt werden können, beispielsweise von -50 °C bis ca. 250 °C, vorzugsweise von Raumtemperatur bis ca. 100 °C.

Darüber hinaus kann die Messeinrichtung nach Fig. 4 eine Steuerung 10 und eine Kommunikationsschnittstelle 11 aufweisen, wobei die Steuerung 10 mit mindestens einer der Komponenten der Messeinrichtung 2 elektronisch verbunden ist. Über die Steuerung 10 sind beispielsweise die Temperierelemente 12 entsprechend dem angewendeten thermischen Analyseverfahren und/oder die Kommunikation mit der Auswerteeinrichtung 5 über die Kommunikationsschnittstelle 11 steuerbar.

In der vorangegangenen detaillierten Beschreibung sind verschiedene Merkmale zur Verbesserung der Stringenz der Darstellung in einem oder mehreren Beispielen zusammengefasst worden. Es sollte dabei jedoch klar sein, dass die obige Beschreibung lediglich illustrativer, keinesfalls jedoch beschränkender Natur ist.

Die Ausführungsbeispiele wurden ausgewählt und beschrieben, um die der Erfindung zugrundeliegenden Prinzipien und ihre Anwendungsmöglichkeiten in der Praxis bestmöglich darstellen zu können. Dadurch können Fachleute die Erfindung und ihre verschiedenen Ausführungsbeispiele in Bezug auf den beabsichtigten Einsatzzweck optimal modifizieren und nutzen. **In** den Ansprüchen sowie der Beschreibung werden die Begriffe "beinhaltend" und "aufweisend" als neutralsprachliche Begrifflichkeiten für die entsprechenden Begriffe "umfassend" verwendet. Weiterhin soll eine Verwendung der Begriffe "ein", "einer" und "eine" eine Mehrzahl derartig beschriebener Merkmale und Komponenten nicht grundsätzlich ausschließen.

### Bezugszeichenliste

- 1: System
- 2: Messeinrichtung
- 3: Sensor
- 4: Probe
- 5: Auswerteeinrichtung
- 6a, 6b: erste, zweite Anwendungssoftwareinstanz
- 7: Display
- 8: Kommunikationsschnittstelle
- 9: externer Kommunikationsteilnehmer
- 10: Steuerung
- 11: Kommunikationsschnittstelle der Messeinrichtung
- 12: Temperierelemente
- 13: Referenz
- 14: Behälter

- V1: Einbringen einer Probe
- V2: Erfassen von Messwerten
- V3: Senden der Messwerte
- V3.1: Koppeln der Auswerteeinrichtung mit einem Datenverarbeitungsgerät
- V3.2: Koppeln der Auswerteeinrichtung mit diagnostischen Geräten
- V4: Abschätzen eines Gesundheitszustandes
- V5: Visualisieren oder Audiovisualisieren
- V6: Steuern kommunikationsfähiger Gebäudeeinrichtungen
- VM: vergleichbare Messwerte anderer Patienten
- VP: Vitalparameter

## Patentansprüche

1. Verfahren zur Analyse durch dynamische Differenzkalorimetrie (DSC) von biologischem Material, insbesondere Blut, Urin, Schweiß oder Hautgewebe, mit den Schritten:
Einbringen (V1) einer Probe (4) mit biologischem Material eines Patienten in einen Sensor (3) einer Messeinrichtung (2);
Erfassen (V2) von Messwerten durch die Messeinrichtung (2);
Senden (V3) der Messwerte an eine Auswerteeinrichtung (5), die mit der Messeinrichtung (2) kommuniziert;
Abschätzen (V4) eines Gesundheitszustandes des Patienten anhand von den Gesundheitszustand charakterisierenden Datenstrukturen auf Basis der Messwerte mittels der Auswerteeinrichtung (5), auf der eine erste Anwendungssoftwareinstanz (6a) ausgeführt wird, wobei der Sensor (3) nach dem Schritt des Abschätzens (V4) entsorgt wird, wobei beim Abschätzen (V4) des Gesundheitszustandes zusätzlich zu den Messwerten ferner Datensätze mit medizinischen Informationen berücksichtigt werden, wobei die Datensätze vergangene medizinische Informationen des Patienten oder Daten aus anderen Messmethoden, die der Patient zusätzlich zu diesem Verfahren anwendet, sind, wobei die Datensätze ferner vergleichbare Messwerte (VM) anderer Patienten und auf die vergleichbaren Messwerte angewendete Behandlungsmaßnahmen umfassen, und durch kommunikatives Koppeln (V3.1) der Auswerteeinrichtung (5) mit einem Datenverarbeitungsgerät die Datensätze an die Auswerteeinrichtung (5) übermittelt werden; und
Visualisieren oder Audiovisualisieren (V5) des Gesundheitszustandes auf einem Display (7).

2. Verfahren nach Anspruch 1, wobei die Datensätze Vitalparameter (VP) des Patienten, insbesondere Blutzuckergehalt, Blutdruck, Herzfrequenz und ähnliches, umfassen, welche durch kommunikatives Koppeln (V3.2) der Auswerteeinrichtung (5) mit diagnostischen Geräten, insbesondere Blutzucker-/Blutdruckmessgeräten oder ähnlichen, und/oder Computer-Hardware, insbesondere Fitness-Trackern, an die Auswerteeinrichtung (5) übermittelt werden, wobei die Vitalparameter (VP) im Wesentlichen zu derselben Zeit wie die Probe (4) mit biologischem Material erhoben werden.

3. Verfahren nach wenigstens einem der vorhergehenden Ansprüche, wobei die Messeinrichtung (2) ein weiteres thermisches Analyseverfahren zur Messung von abgegebener oder aufgenommener Wärmemenge des biologischen Materials während eines thermischen Prozesses durchführt.

4. Verfahren nach wenigstens einem der vorhergehenden Ansprüche, wobei die Auswerteeinrichtung (5) mit einer Vorrichtung (9), welche vorzugsweise als Spiegel, Fernseher und/oder Computer-Hardware, insbesondere PC, Smartphone, Smartwatch und/oder Fitness-Tracker, ausgebildet ist, über jeweilige Kommunikationsschnittstellen kommuniziert.

5. Verfahren nach wenigstens einem der vorhergehenden Ansprüche, wobei das Verfahren ferner einen Schritt des Steuerns (V6) kommunikationsfähiger Gebäudeeinrichtungen, insbesondere Heizungen, Gebäudebelüftungen und/oder Wecker, zur Unterstützung von Behandlungsmaßnahmen für den Patienten in Abhängigkeit von dem abgeschätzten Gesundheitszustand umfasst.

6. System (1) zur Analyse durch dynamische Differenzkalorimetrie (DSC) von biologischem Material, insbesondere Blut, Urin, Schweiß oder Hautgewebe, mit:
einer Messeinrichtung (2), welche einen Sensor (3) aufweist, der eine Probe (4) mit biologischem Material eines Patienten beinhaltet, und dazu ausgebildet ist, Messwerte an eine Auswerteeinrichtung (5) zu senden, wobei der Sensor (3) ein Einwegprodukt ist;
der Auswerteeinrichtung (5), welche eine erste Anwendungssoftwareinstanz (6a) aufweist und dazu ausgebildet ist, die Messwerte zu empfangen und einen Gesundheitszustand des Patienten anhand von den Gesundheitszustand charakterisierenden Datenstrukturen auf Basis der Messwerte und auf Basis von Datensätzen mit medizinischen Informationen abzuschätzen, wobei die Datensätze vergangene medizinische Informationen des Patienten oder Daten aus anderen Messmethoden, die der Patient zusätzlich zu der dynamischen Differenzkalorimetrie anwendet, sind, wobei die Datensätze ferner vergleichbare Messwerte (VM) anderer Patienten und auf die vergleichbaren Messwerte angewendete Behandlungsmaßnahmen umfassen, und wobei die Auswerteeinrichtung (5) mit einem Datenverarbeitungsgerät zum Übermitteln der Datensätze an die Auswerteeinrichtung (5) kommunikativ koppelbar ist; und
einem Display (7), welches dazu ausgebildet ist, den Gesundheitszustand zu visualisieren oder audiovisualisieren.

7. System (1) nach Anspruch 6, wobei die Messeinrichtung (2) ferner dazu ausgebildet ist, ein weiteres thermisches Analyseverfahren zur Messung von abgegebener oder aufgenommener Wärmemenge des biologischen Materials während eines thermischen Prozesses zu ermöglichen.

8. System (1) nach Anspruch 6 oder 7, wobei die Messeinrichtung (2) ein Einwegprodukt ist.

9. System (1) nach wenigstens einem der Ansprüche 6 bis 8, wobei die Messeinrichtung (2), die Auswerteeinrichtung (5) und das Display (7) von einem Gehäuse, insbesondere einem gemeinsamen Gehäuse, zumindest teilweise umgeben sind.

## Claims

1. Process for analysing biological material by dynamic differential calorimetry (DSC), in particular blood, urine, sweat or skin tissue, comprising the steps of:
introducing (V1) a sample (4) with biological material from a patient into a sensor (3) of a measurement device (2);
detecting (V2) measurement values by way of the measurement device (2);
sending (V3) the measurement values to an evaluation device (5) which communicates with the measurement device (2);
estimating (V4) a state of health of the patient from data structures which characterise the state of health, on the basis of the measurement values, by means of the evaluation device (5), on which a first application software instance (6a) is executed, the sensor (3) being disposed of after the estimation step (V4), further datasets which contain medical information being taken into account, in addition to the measurement values, in estimating (V4) the state of health, the datasets comprising past medical information from the patient or data from other measurement methods which the patient uses in addition to this process, the datasets further comprising comparable measurement values (VM) from other patients and processing measures applied to the comparable measurement values, and the datasets being transmitted to the evaluation device (5) by communicatively coupling (V3.1) the evaluation device (5) to a data processing device; and
visualising or audiovisualising (V5) the state of health on a display (7).

2. Process according to claim 1, wherein the datasets comprise vital parameters (VP) of the patient, in particular blood sugar level, blood pressure, heartrate and the like, which are transmitted to the evaluation device (5) by communicatively coupling (V3.2) the evaluation device (5) to diagnostic devices, in particular blood sugar/blood pressure measurement devices or the like, and/or computer hardware, in particular fitness trackers, the vital parameters (VP) being collected substantially at the same time as the sample (4) with biological material.

3. Process according to at least one of the preceding claims, wherein the measurement device (2) carries out a further thermal analysis process for measuring the amount of heat released or absorbed by the biological material during a thermal process.

4. Process according to at least one of the preceding claims, wherein the evaluation device (5) communicates with a device (9), which is preferably configured as a mirror, a television and/or computer hardware, in particular a PC, smartphone, smartwatch and/or fitness tracker, via respective communication interfaces.

5. Process according to at least one of the preceding claims, wherein the process further comprises a step of controlling (V6) communication-capable building devices, in particular heating systems, building ventilation systems and/or alarm clocks, to support treatment measures for the patient as a function of the estimated state of health.

6. System (1) for analysing biological material by dynamic differential calorimetry (DSC), in particular blood, urine, sweat or skin tissue, comprising:
a measurement device (2) which has a sensor (3) containing a sample (4) with biological material from a patient and is configured to send measurement values to an evaluation device (5), the sensor (3) being a disposable product;
the evaluation device (5), which has a first application software instance (6a) and is configured to receive the measurement values and to estimate a state of health of the patient from data structures which characterise the state of health, on the basis of the measurement values and on the basis of datasets comprising medical information, the datasets comprising past medical information from the patient or data from other measurement methods which the patient uses in addition to dynamic differential calorimetry, the datasets further comprising comparable measurement values (VM) from other patients and processing measures applied to the comparable measurement values, and the evaluation device (5) being communicatively couplable to a data processing device to transmit the datasets to the evaluation device (5); and
a display (7) configured to visualise or audiovisualise the state of health.

7. System (1) according to claim 6, wherein the measurement device (2) is further configured to make possible a further thermal analysis process for measuring the amount of heat released or absorbed by the biological material during a thermal process.

8. System (1) according to either claim 6 or claim 7, wherein the measurement device (2) is a disposable product.

9. System (1) according to at least one of claims 6 to 8, wherein the measurement device (2), the evaluation device (5) and the display (7) are surrounded at least in part by a housing, in particular a shared housing.

## Revendications

1. Procédé d'analyse par calorimétrie différentielle à balayage (DSC) dynamique de matériel biologique, en particulier de sang, d'urine, de sueur ou de tissu cutané, comprenant les étapes suivantes :
introduction (V1) d'un échantillon (4) de matériel biologique d'un patient dans un capteur (3) d'un dispositif de mesure (2) ;
détection (V2) de valeurs de mesure par le dispositif de mesure (2) ;
envoi (V3) des valeurs de mesure à un dispositif d'évaluation (5) qui communique avec le dispositif de mesure (2) ;
estimation (V4) de l'état de santé du patient à l'aide de structures de données caractérisant l'état de santé sur la base des valeurs de mesure au moyen du dispositif d'évaluation (5) sur lequel est exécutée une première instance de logiciel d'application (6a), le capteur (3) étant éliminé après l'étape d'estimation (V4), des ensembles de données contenant des informations médicales étant pris en compte en plus des valeurs de mesure lors de l'estimation (V4) de l'état de santé, les ensembles de données étant des informations médicales passées du patient ou des données provenant d'autres méthodes de mesure que le patient utilise en plus du présent procédé, les ensembles de données comprenant en outre des valeurs de mesure comparables (VM) d'autres patients et des mesures thérapeutiques appliquées aux valeurs de mesure comparables, et les ensembles de données étant transmis au dispositif d'évaluation (5) par couplage communicatif (V3.1) du dispositif d'évaluation (5) avec un appareil de traitement de données ; et
visualisation ou audiovisualisation (V5) de l'état de santé sur un écran (7).

2. Procédé selon la revendication 1, dans lequel les ensembles de données comprennent des paramètres vitaux (VP) du patient, en particulier la glycémie, la pression artérielle, la fréquence cardiaque et analogues, qui sont transmis par couplage communicatif (V3.2) du dispositif d'évaluation (5) avec des appareils de diagnostic, en particulier des appareils de mesure de la glycémie/pression artérielle ou analogues et/ou du matériel informatique, en particulier des trackers d'activité physique, au dispositif d'évaluation (5), les paramètres vitaux (VP) étant recueillis essentiellement au même moment que l'échantillon (4) de matériel biologique.

3. Procédé selon au moins l'une des revendications précédentes, dans lequel le dispositif de mesure (2) effectue un autre procédé d'analyse thermique pour mesurer la quantité de chaleur dégagée ou absorbée par le matériel biologique pendant un processus thermique.

4. Procédé selon au moins l'une des revendications précédentes, dans lequel le dispositif d'évaluation (5) communique avec un dispositif (9), qui est de préférence conçu comme un miroir, un téléviseur et/ou un matériel informatique, en particulier un PC, un smartphone, une smartwatch et/ou un tracker d'activité physique, via des interfaces de communication respectives.

5. Procédé selon au moins l'une des revendications précédentes, le procédé comprenant en outre une étape de commande (V6) d'équipements de bâtiment communicants, en particulier de systèmes de chauffage, de ventilation de bâtiment et/ou de réveils, afin de soutenir les mesures thérapeutiques pour le patient en fonction de l'état de santé estimé.

6. Système (1) pour l'analyse par calorimétrie différentielle à balayage (DSC) dynamique de matériel biologique, en particulier de sang, d'urine, de sueur ou de tissu cutané, comprenant :
un dispositif de mesure (2) qui présente un capteur (3) contenant un échantillon (4) de matériel biologique d'un patient et qui est conçu pour envoyer des valeurs de mesure à un dispositif d'évaluation (5), le capteur (3) étant un produit jetable ;
le dispositif d'évaluation (5) qui présente une première instance de logiciel d'application (6a) et est conçu pour recevoir les valeurs de mesure et évaluer l'état de santé du patient à l'aide de structures de données caractérisant l'état de santé sur la base des valeurs de mesure et sur la base d'ensembles de données contenant des informations médicales, les ensembles de données étant des informations médicales passées du patient ou des données provenant d'autres méthodes de mesure que le patient utilise en plus de la calorimétrie différentielle dynamique, les ensembles de données comprenant en outre des valeurs de mesure comparables (VM) d'autres patients et des mesures thérapeutiques appliquées aux valeurs de mesure comparables, et le dispositif d'évaluation (5) pouvant être couplé de manière communicative à un appareil de traitement de données pour transmettre les ensembles de données au dispositif d'évaluation (5) ; et
un écran (7) conçu pour visualiser ou audiovisualiser l'état de santé.

7. Système (1) selon la revendication 6, dans lequel le dispositif de mesure (2) est en outre conçu pour permettre un autre procédé d'analyse thermique pour mesurer la quantité de chaleur dégagée ou absorbée par le matériau biologique pendant un processus thermique.

8. Système (1) selon la revendication 6 ou 7, dans lequel le dispositif de mesure (2) est un produit jetable.

9. Système (1) selon au moins l'une des revendications 6 à 8, dans lequel le dispositif de mesure (2), le dispositif d'évaluation (5) et l'écran (7) sont au moins partiellement entourés par un boîtier, en particulier un boîtier commun.
